# EUROPEAN PATENT SPECIFICATION

(11) **EP 2 068 774 B1**
(45) Date of publication and mention of the grant of the patent: **01.04.2015**
(21) Application number: 07842877.8
(22) Date of filing: 20.09.2007
(51) Int. Cl.: A61F 2/46

(54) **NUCLEUS PULPOSUS INJECTION DEVICES**
VORRICHTUNGEN ZU INJEKTION VON NUCLEUS PULPOSUS
DISPOSITIFS D'INJECTION DE NUCLEUS PULPOSUS

(30) Priority: 03.10.2006 US 538193
(43) Date of publication of application: 17.06.2009
(73) Proprietor: Depuy Spine, Inc., Raynham, MA 02767 (US)
(72) Inventor: REYNOLDS, Martin A., Mansfield, Massachusetts 02048 (US)
(74) Representative: Orr, Robert
(86) International application number: PCT/US2007/079022
(87) International publication number: WO 2008/042612

(56) References cited:
- EP-A- 1 495 730
- WO-A-2004/014262
- WO-A-2006/047356
- FR-A- 2 629 337
- US-A1- 2006 264 966

## Description

### BACKGROUND OF THE INVENTION

### 1. Field of the Invention

This invention is concerned with repair of intervertebral discs, particularly with injectable materials for repair of the nucleus pulposus of an intervertebral disc.

### 2. Related Art

When considering the use of in-situ curing polymers for nucleus pulposus replacement, sealing the annulus fibrosus to contain the injected material both during the filling procedure and in the long term is a problem. Existing concepts for closing the annulus rely on elaborate anchoring means and multiple materials making the surgical technique difficult and raising questions as to biocompatibility and the long term survivability of a multi-part construct. Also, the annulotomy required for discectomy and cavity filling can become a site of Functional Spinal Unit (FSU) instability due to the focal defect in the annulus.

There are no commercially available annular plugs but there are several patents in this area.

U.S. 6,592,625 (Cauthen) describes repair and reconstruction of the annulus fibrosus by insertion of a collapsible patch which after insertion expands to bridge an aperture in the subannular space of the annulus fibrosus.

U.S. 6,425,919 (Lambrecht) discloses a disc herniation constraining device which can include the insertion of an augmentation material within the disc. The constraining device can include a support member for support of a herniated portion of a disc. A defect in the annulus of a disc can be closed using a prosthesis such as a barrier.

EP 1495730 (Lin) discloses a spine filling device which comprises a filling member and a pasty medicine. The filling member is formed of a holding portion and an injection port which are integrally formed by a flexible and permeable wall having a plurality of pores, each having a diameter smaller than 0.1mm. The filling member is contracted before being implanted in a spinal segment or between two adjacent spinal segments. The pasty medicine is injected into the holding portion via the injection port, thereby resulting in expansion of the filling member.

WO 2004/014262 (DePuy International Limited) discloses an instrument for deploying a bone cement material in a bone cavity, the material being formed from two reactive components which, when mixed, react to form a useable cement. The instrument comprises a chamber in which the components of the material can be mixed, and a mixing tool which extends into the chamber, and which can be manipulated from outside the chamber, to cause the components of the material to mix. Material can be discharged from the chamber after it has mixed through an outlet. A piston, which can be moved through the chamber to apply positive pressure to mixed material within the chamber, can displace the mixed material from the chamber through the outlet into the bone cavity. A sealing component fits over the bone cavity to seal the cavity around the chamber outlet and to minimise leakage of bone cement that has injected into the cavity, and a sensor is provided for measuring the pressure to which the bone cement is subjected during displacement from the chamber.

FR 2629337 (Bigan et. al.) discloses a device for intra-osseus sealing of a prosthesis element, in particular for a joint prosthesis, comprising a stem which is intended to be inserted into a cavity made in the bone, and to be immobilised therein. The stem is at least partially surrounded over its part which is inserted into the bone cavity by a flexible covering forming a deformable leaktight chamber around the said part, with, emerging in said chamber, a channel made in the stem and emerging at its other end beyond the limit of insertion into the bone cavity. After forming the cavity in the bone, a plug is placed in the medullary canal, the stem is inserted into this cavity and the prosthesis is prepositioned by injecting under pressure, through the channel, into the leaktight chamber defined by the covering, a product which can set at a low temperature with a delay of a few minutes.

WO 2006/047356 (Synecor, LLC) discloses devices for access and/or closure of an annulotomy of a subject. The devices can be separate from, joined to, or integral with an artificial disc nucleus, and may comprise any of numerous alternative means for securing the device within an annulotomy. The devices can provide access to a disc nucleus for one or more procedures requiring access and may close an annulotomy following the conclusion of one or more procedures.

### BRIEF DESCRIPTION OF THE DRAWINGS

Figs. 1a-c depict a problem and solution for use of in-situ curing polymers for nucleus replacement.
Figs. 2a-b depict two embodiments of an injection port forming part of a kit of the present invention.
Figs. 3a-c depict ways of deploying injection ports.
Fig. 4 describes an added feature that prevents backout of injection ports.
Fig. 5 depicts use of an elongated flexible needle in conjunction with the invention.
Figs. 6a-d depict alternative embodiments of injection ports forming part of a kit of the present invention.
Figs. 7a-b depict a fully in-situ cured nucleus replacement.
Figs. 8a-c describe a problem and solutions to protruding nucleus material under load.

### SUMMARY OF THE INVENTION

The present invention provides a kit as claimed in claim 1.

Further features of the kit are defined in the dependent claims.

The present invention may have a utility in a method for repair of an intervertrebral disc. The method does not fall within the scope of the claimed invention. The method comprises the steps of:
a) providing an injection port formed of a material capable of being injected;
b) inserting the injection port into a defect of the annulus fibrosus of the intervertebral disc; and
c) injecting an injectable in-situ curable form of the injection port material into a defect of the nucleus pulposus of the invertebral disc.

### DETAILED DESCRIPTION OF PREFERRED EMBODIMENTS OF THE INVENTION

The advantages of the present invention include sealing the annulus during injection of in-situ curing polymer nuclear replacement materials to prevent leakage during filling and to provide an aid to achieve complete nuclear filling; providing a plug that does not require secondary anchoring means to enable an easier surgical technique; providing a plug of the same or substantially the same material of an in-situ curable biocompatible polymer minimizes concern over debris generation normally associated with constructs made of multiple components; providing a plug that is made of the same material as the injectable nucleus material so as to minimize the need to characterize the biocompatibility of multiple materials and to provide reliable chemical bonding between the injected nucleus and the plug to essentially construct a unitary nuclear/annular implant device; and providing structural stability to the focal location of the annulotomy.

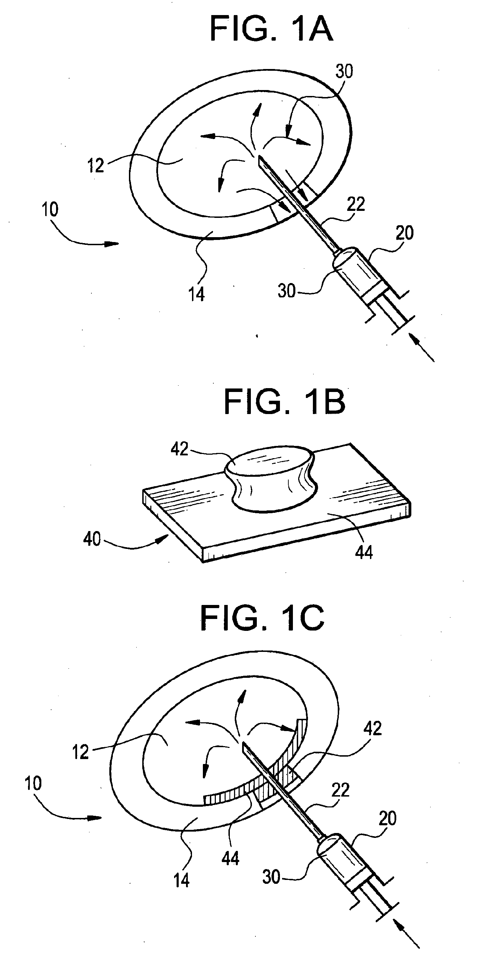

When considering the use of in-situ curing biocompatible polymers for nucleus replacement, sealing the annulus to contain the injected material both during the procedure, and/or in the long term is a problem. Fig. 1a depicts a rather exaggerated representation of this problem where nuclear replacement material 30 is injected into the nucleus 12 of disc 10 and is dispensed from syringe 20 and exits disc 10 around the gaps between the needle 22 of syringe 20 and annulus fibrosus 14.

One solution to the foregoing problem is depicted in Figs. 1b and 1c. Particularly this embodiment of the invention solves the problem by providing an injection port (such as an elastomer septum or annular plug) that serves as a "limiter" to the amount of material that can be injected, a guard against leakage during injection, and, serves as an annulus plug long term. The plug portion, in addition to sealing the annulus may also help structurally fortify the annulus at the location of the annulotomy.

More particularly, one embodiment of injection port 40 is shown to comprise plug 42 and wing(s) 44.

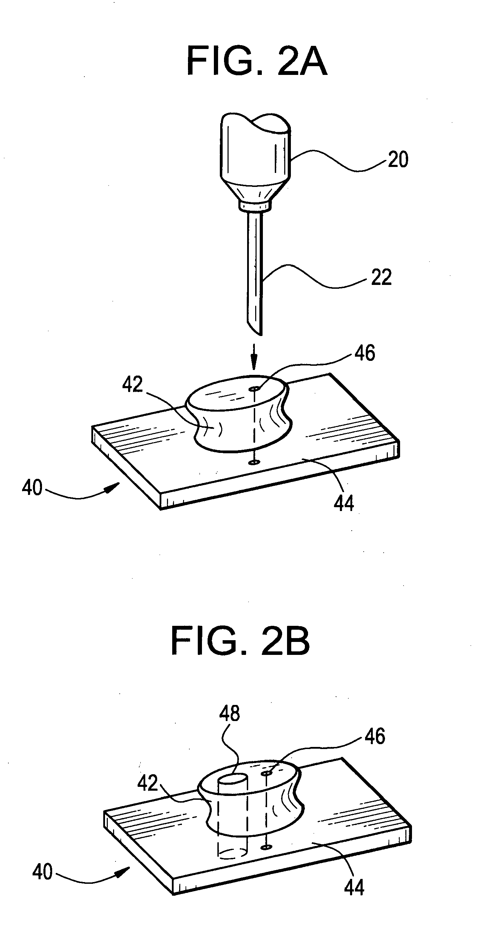

Figs. 2a and 2b show further embodiments of injection port 40. In Fig. 2a, injection port 40 comprizes preformed passage 46 to help enable needle 22 of syringe 20 to pass through injection port 40. Fig. 2b depicts yet another embodiment wherein vent 48 is incorporated. Vent 48 is preformed and aids in complete cavity filling (limits air or other fluid entrapment) and to provide visual indication of complete fill as well.

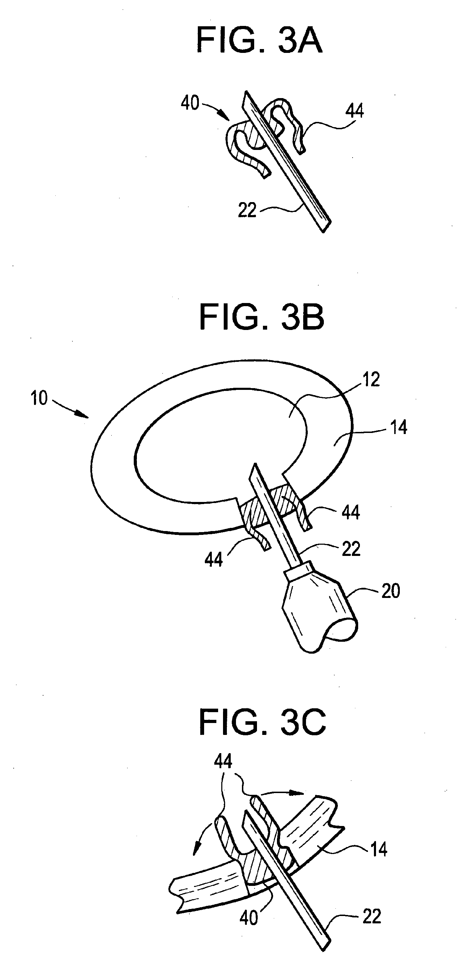

In use as shown in Fig. 3, the injection port 40 is placed on needle 22 of syringe 20 prior to entering annulus fibrosus 14 and nucleus 12 of disc 10. Injection port 40 would be a solid piece to be pierced by the needle intraoperatively or pre-pierced in a kit with the needle. Fig. 3 actually shows two ways of deployment for injection ports having wings; one with wings 44 in a "back deployment" mode as shown in Figs. 3 (a) and 3(b) and another with wings 44 in a "forward deployment" mode as shown in Fig. 3(c). Once inserted and material injected, the syringe is withdrawn from the injection port.

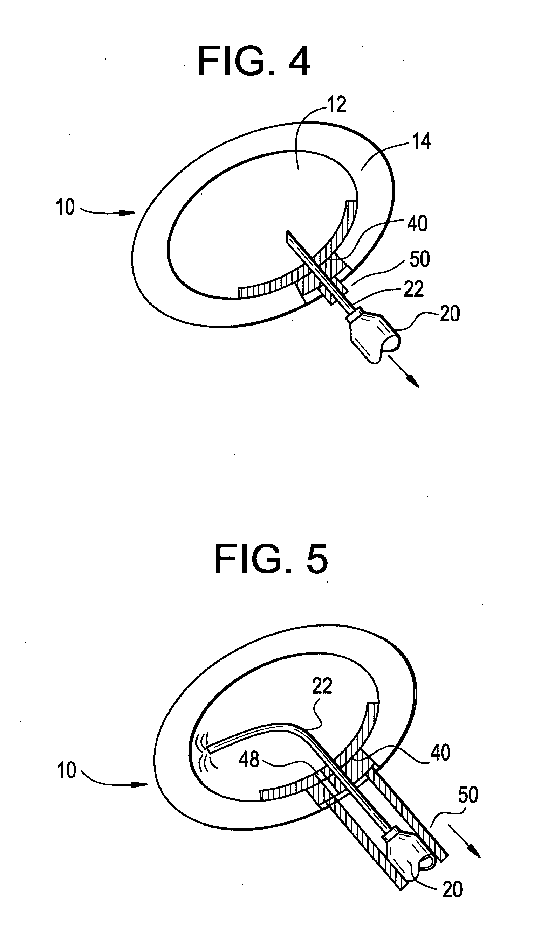

In some embodiments, it may be useful to equip the syringe with a "stabilizer" for avoiding pulling the injection port out during the time the needle of the syringe is withdrawn from the injection port. As used herein, the term "stabilizer" is intended to describe a feature that prevents backout of the injection port from the disc once the needle of the syringe is withdrawn. As depicted in Fig. 4, the stabilizer is depicted as tube 50 through which passes needle 22 of syringe 20. When the syringe is drawn away from injection port 40, stabilizer 50 is held against injection port 40 as needle 22 is withdrawn thereby preventing backout of injection port 40.

Fig. 5 depicts yet another embodiment of this invention wherein needle 22 is shown to be of an elongated, flexible type, suitable to more precise placement of intradiscal material than standard straight needles. Fig. 5 also shows optional vent 48.

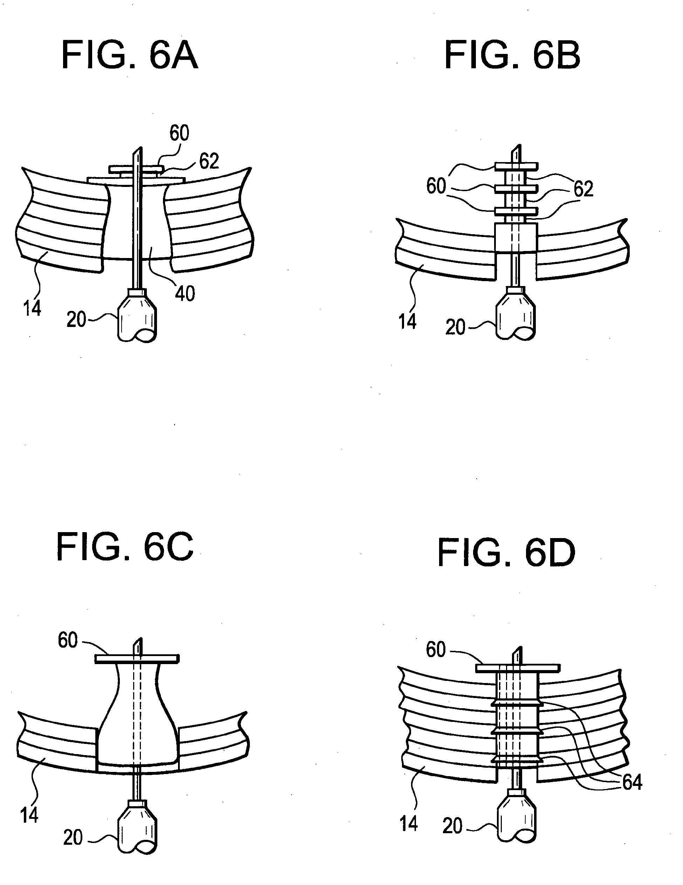

Figs. 6a to 6d depict various embodiments for injection port 40. Fig. 6a shows injection port 40 with raised projection 60 with accompanying undercut 62 which provides additional bonding surface area plus mechanical interlock to help prevent injection port extrusion.

Fig. 6b depicts injection port 40 with a multiple projection 60 design. Fig. 6c shows an alternate projection embodiment for injection port 40.

Finally Fig. 6d shows injection port 40 with projection 60 and multiple barbs 64 for securement to the annulus fibrosus 14. The barbs help to stabilize injection port 40 both during injection and needle removal. It should be noted that injection ports 40 depicted in Figs. 6b and 6c may have a loose, slip, or press fit and do not necessarily require flaps or wings 44.

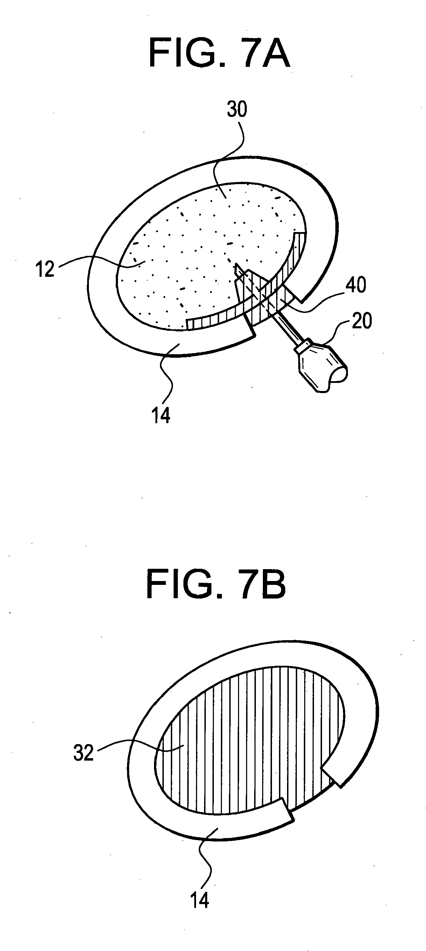

Thus when the in-situ curable polymer 30 is cured and bonded to injection port 40, uniform cured nucleus replacement 32 is formed as depicted in Figs. 7a and 7b. Laboratory testing has shown that material residing within the annular wall may intermittently protrude beyond the confines of the annular periphery under flexural loading. Therefore, it may be advisable to decrease the height of the injection port as it resides within the annular defect, or, use the injection port in a purely internal configuration.

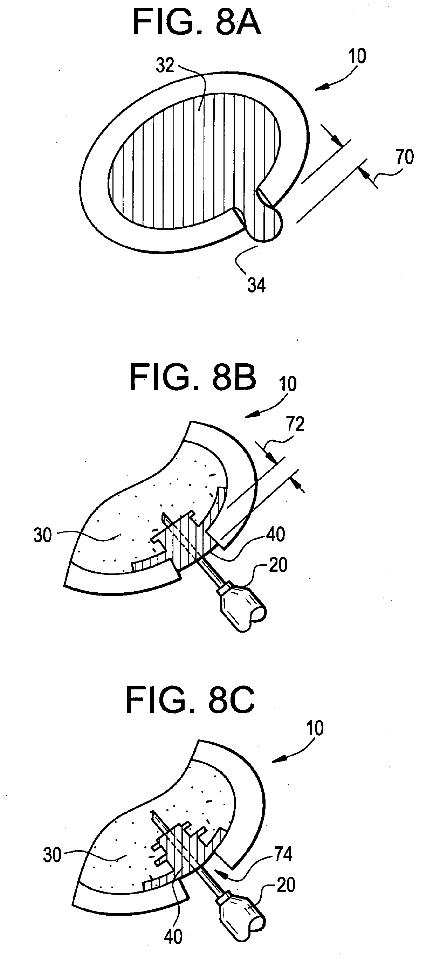

Figs. 8a to 8c depict two solutions to a potential problem should cured nucleus replacement material 32 intermittently protrude beyond the confines of the annular periphery under flexural loading. More specifically, Fig. 8a shows fully cured nucleus replacement 32 with a portion of 32 (shown as 34) protruding the annular wall of disc 10 by a distance of 70. Fig. 8b shows one way to prevent or minimize protrusion 34 by using injection port 40 that has a shortened annular portion that is designed to a dimension not to protrude the annular wall of disc 10 under load. Alternatively, Fig. 8c shows injection port 40 which is completely within the annular wall of disc 10 such that void 74 results.

The in-situ curing polymer nuclear replacement material 30 is the same or substantially the same as injection port 40. Thus, having injection port 40, pre-manufactured of the same or substantially the same material as the injected in-situ curable nuclear replacement material 30, will result in an intimate chemical bond between injection port 40 and nuclear replacement material 30 and yield essentially a final construct of unitary construction. The benefits of such a construction includes: (i) greater resistance of injection port expulsion due to the bonding of the injection port with the nuclear replacement material; (ii) only needing biocompatibility data for one material; (iii) elimination/minimization over possible material reaction between two dissimilar components; and (iv) elimination/minimization of relative micro motion between the port component and nucleus replacement component and the production of associated wear debris.

The term "substantially the same material" encompasses materials that are not identical in composition to the first material being referred to and indicates materials that have similar or like properties with reference to the properties of the first referenced material. In particular, a key property that both materials should possess is the same durometer, which is the material's hardness, particularly the material's resistance to permanent indentation. This is a key property in the setting of a material useful for application as an intervertebral disc.

Desirably, injection port 40 and nuclear replacement material 30 are made of materials capable of polymerizing to form a solid or semi-solid (e.g., gel) like mass, and preferably is capable of being administered to a patient as a liquid, or gel-like material, and then capable of curing inside the patient's body to form the nucleus pulposus implant. Nucleus replacement material 30 may be a monomer, oligomer, or material capable of undergoing cross-linking either by itself, or with the aid of cross-linking agents or external force (e.g., heat, light, water, etc.). Self-curing polymerizable materials include epoxy materials, polyisobutylene rubbers, and other self-cross linking polymers known to those skilled in the art. Moreover, one who is skilled in the art will recognize that the state in which the polymerizable material is used for purposes of this invention may be chosen to correspond with the particular conditions expected during disc reconstruction or repair.

Preferably, the material 30 is a liquid that can be readily injected into the patient, and then cures in a relatively short period of time inside the patient. A preferred polymerizable material used with the invention comprises, or alternatively consists of, a prepolymer component that is admixed with a curative agent prior to administration to the patient, and the admixture of prepolymer and curative agent in situ to produce the nucleus pulposus implant.

U.S. 6,520,992 shows that on self-polymerization, polydimethylsiloxane-based compositions produce silicones which possess both properties. Since the intervertebral disc is subjected to considerable pressure loads, this result was surprising and not predictable. The permanently elastic silicones produced from polydimethylsiloxanes have no deleterious effect on the surrounding tissue, on cell proliferation and on normal cell division. Degenerative changes were not observed. Linear polydimethylsiloxane-based compositions are particularly advantageous.

By copolymerising the methylsiloxane with differently substituted siloxanes, the reaction period for self-polymerization and the properties of the silicone produced can be varied. As an example, instead of methyl groups, phenyl residues, ethyl groups or vinyl residues can be included, to produce mixed methylphenyl-, ethylmethyl- or methylvinyl-siloxanes, for example. Such co-polymerisates can have their substituents randomly arranged or arranged as a block copolymer. The end groups of the polydimethylsiloxane are preferably trimethylsiloxy groups, but at least a portion thereof can be silanol groups, vinyl groups or hydride groups. The viscosity of the linear polydimethylsiloxane can be governed by chain breaking groups, essential for the invention as for use, the compositions must be fluid or pasty.

In order to be self-polymerisable at ambient temperature, the compositions of the invention comprise a catalyst. Known siloxane catalysts are amines, such as aminopropylsilane derivatives, and lead, tin and zinc carbonic acid salts, also organic iron, cadmium, barium, antimony or zirconium salts. Tin octoate, laurate and oleate as well as dibutyltin salts are particularly suitable. The selected catalysts must be biologically compatible. These include addition catalysing noble metal complexes from group VIII, such as platinum, rhodium or ruthenium, which catalyse self-polymerization within suitable polymerization periods in very small amounts, for example concentrations as low as 1 to 2 ppm. In particular, platinum catalysts such as platinum-olefin complexes can catalyse addition of Si--H end groups to olefins such as vinyl functional siloxanes. Preferred compositions which polymerise to permanently elastic silicones are produced from mixtures of linear hydride functional polydimethylsiloxanes and linear vinyl functional polydimethylsiloxanes mixed with a suitable catalyst, in particular a platinum catalyst such as chloroplatinic acid or another platinum compound. In order to avoid premature polyaddition, such compositions are stored in two separate packs and are only combined immediately prior to use. Normally, the catalyst is stored together with the vinyl functional polydimethylsiloxane in one package and the hydride functional polydimethylsiloxane, optionally with the usual hardeners, if necessary with the addition of a vinyl functional polydimethylsiloxane but without the catalyst, is stored in the second package. The ratios of the amounts of both polydimethylsiloxanes used depends on the desired properties of the permanently elastic silicone.

Other useful in situ curable materials include formulations of elastomers/hydrogels such as polyvinylacetate (PVA); polyvinylpyrrolidone (PVP); polyvinylfluoride (PVF); polyethyleneglycol (PEG); carboxymethylcellulose (CMC); hydroxyethylmethacrylate (HEMA); protein polymers; and combinations thereof.

The invention provides a kit comprising sterile components of injection port 40, an injection device such as a syringe, and the in-situ curable injectable material 30. As would be apparent to one of skill in the art, the foregoing components may be comprised of any of the materials hereinbefore disclosed as suitable for use as the appropriate component.

It should be understood that the foregoing disclosure and description of the present invention are illustrative and explanatory thereof and various changes in the size, shape and materials as well as in the description of the preferred embodiment may be made without departing from the scope of the invention as defined by the claims.

## Claims

1. A kit comprising sterile components of an injection port (40), a syringe, and an in-situ curable injectable material (30);
**characterised in that** the injection port and the injectable material comprise the same material.

2. The kit of claim 1, wherein the injection port comprises wings (44).

3. The kit of claim 1, wherein the injection port comprises a projection (60).

4. The kit of claim 1, wherein the injection port comprises a vent (48).

5. The kit of claim 1, wherein the injection port comprises barbs (64).

6. The kit of claim 1, wherein the in-situ curable injection material and the material of which the injection port is made of is selected from the group consisting of epoxy materials, polyisobutylene rubbers, elastomers, hydrogels and mixtures thereof.

7. The kit of claim 1, wherein both the in-situ curable injection material and the material of which the injection port is made of comprises an organosiloxane-based composition.

8. The kit of claim 7, wherein both the in-situ curable injection material and the material of which the injection port is made of comprises a polydimethylsiloxane-based composition.

9. The kit of claim 8, wherein both the in-situ curable injection material and the material of which the injection port is made of comprises a linear polydimethylsiloxane-based composition.

10. The kit of claim 1, wherein both the in-situ curable injection material and the material of which the injection port is made of is selected from the group consisting of polyvinylacetate (PVA); polyvinylpyrrolidone (PVP); polyvinylfluoride (PVF); polyethyleneglycol (PEG); carboxymethylcellulose (CMC); hydroxyethylmethacrylate (HEMA); protein polymers; and combinations thereof.

11. A kit comprising sterile components of an injection port (40), a syringe, and an in-situ curable injectable material (30);
**characterised in that** the injection port is of a material having a hardness which is the same as a hardness of the injectable material.

## Patentansprüche

1. Satz umfassend sterile Komponenten eines Einspritzkanals (40), eine Spritze und in-situ vernetzbares einspritzbares Material (30);
**dadurch gekennzeichnet, dass** der Einspritzkanal und das einspritzbare Material das gleiche Material umfassen.

2. Satz nach Anspruch 1, wobei der Einspritzkanal Flügel (44) umfasst.

3. Satz nach Anspruch 1, wobei der Einspritzkanal einen Vorsprung (60) umfasst.

4. Satz nach Anspruch 1, wobei der Einspritzkanal ein Luftloch (48) umfasst.

5. Satz nach Anspruch 1, wobei der Einspritzkanal Widerhaken (64) umfasst.

6. Satz nach Anspruch 1, wobei das in-situ vernetzbare Einspritzmaterial und das Material aus dem der Einspritzkanal hergestellt ist, aus der Gruppe bestehend aus Epoxymaterialien, Polysobutylen-Gummis, Elastomere, Hydrogele und deren Mischungen ausgewählt ist.

7. Satz nach Anspruch 1, wobei sowohl das in-situ vernetzbare Einspritzmaterial als auch das Material aus dem der Einspritzkanal hergestellt ist eine Organosiloxan-basierte Zusammensetzung umfasst.

8. Satz nach Anspruch 7, wobei sowohl das in-situ vernetzbare Einspritzmaterial als auch das Material aus dem der Einspritzkanal hergestellt ist eine Polydimethylsiloxan-basierte Zusammensetzung umfasst.

9. Satz nach Anspruch 8, wobei sowohl das in-situ vernetzbare Einspritzmaterial als auch das Material aus dem der Einspritzkanal hergestellt ist, eine lineare Polydimethylsiloxan-basierte Zusammensetzung umfasst.

10. Satz nach Anspruch 1, wobei sowohl das in-situ vernetzbare Einspritzmaterial als auch das Material aus der Gruppe bestehend aus Polyvinylacetat (PVA); Polyvinylpyrrolidon (PVP); Polyvinylfluorid (PVF); Polyethylenglycol (PEG); Carboxymethylcellulose (CMC); Hydroxyethylmethacrylat (HEMA); Proteinpolymeren; und Kombainationen daraus ausgewählt ist.

11. Satz umfassend sterile Komponenten eines Injektionskanals (40), eine Spritze und in-situ vernetzbares einspritzbares Material (30);
**dadurch gekennzeichnet, dass** der Einspritzkanal aus einem Material hergestellt ist, welcher eine Härte aufweist, die gleich ist zu einer Härte des einspritzbaren Materials.

## Revendications

1. Kit comprenant des composants stériles d'un orifice d'injection (40), une seringue, et un matériau injectable durcissant in-situ (30) ;
**caractérisé en ce que** l'orifice d'injection et le matériau injectable comprennent le même matériau.

2. Kit selon la revendication 1, dans lequel l'orifice d'injection comprend des ailettes (44).

3. Kit selon la revendication 1, dans lequel l'orifice d'injection comprend une saillie (60).

4. Kit selon la revendication 1, dans lequel l'orifice d'injection comprend un évent (48).

5. Kit selon la revendication 1, dans lequel l'orifice d'injection comprend des barbes (64).

6. Kit selon la revendication 1, dans lequel le matériau d'injection durcissant in-situ et le matériau dans lequel l'orifice d'injection est réalisé est sélectionné dans le groupe consistant un matériaux époxy, caoutchoucs poly-iso-butylènes, élastomères, hydrogels et leurs mélanges.

7. Kit selon la revendication 1, dans lequel à la fois le matériau d'injection durcissant in-situ et le matériau dans lequel l'orifice d'injection est réalisé comprend une composition à base d'organo-siloxane.

8. Kit selon la revendication 7, dans lequel à la fois le matériau d'injection durcissant in situ et le matériel dans lequel l'orifice d'injection est réalisé comprend une composition à base de poly-diméthyle-siloxane.

9. Kit selon la revendication 8, dans lequel à la fois le matériau d'injection durcissant in-situ et le matériau dans lequel l'orifice d'injection est réalisé comprend une composition à base de poly-diméthyle-siloxane linéaire.

10. Kit selon la revendication 1, dans lequel à la fois le matériau d'injection durcissant in-situ et le matériau dans lequel l'orifice d'injection est réalisé est sélectionné dans le groupe consistant en polyvinyle-acétate (PVA) ; polyvinyle-pyrrolidone (PVP) ; polyvinyle-fluorure (PVF) ; polyéthylène-glycol (PEG) ; carboxy-méthyle-cellulose (CMC) ; hydroxy-éthyle-méthacrylate (HEMA) ; polymères protéines ; et leurs combinaisons.

11. Kit comprenant des composants stériles d'un orifice d'injection (40), une seringue, et un matériau injectable durcissant in-situ (30) ;
**caractérisé en ce que** l'orifice d'injection est en un matériau d'une dureté qui est la même qu'une dureté du matériau injectable.
